Europäisches Patentamt

European Patent Office

Office européen des brevets

(11) Veröffentlichungsnummer: **0 169 494**
**B1**

# EUROPÄISCHE PATENTSCHRIFT

(45) Veröffentlichungstag der Patentschrift:
**20.04.88**

(51) Int. Cl.⁴: **A 61 M 5/30**

(21) Anmeldenummer: **85108933.4**

(22) Anmeldetag: **17.07.85**

(54) **Nadelloses Injektionsgerät.**

(30) Priorität: **24.07.84 DE 3427187**

(43) Veröffentlichungstag der Anmeldung:
**29.01.86 Patentblatt 86/5**

(45) Bekanntmachung des Hinweises auf die Patenterteilung:
**20.04.88 Patentblatt 88/16**

(84) Benannte Vertragsstaaten:
**CH DE FR GB LI**

(56) Entgegenhaltungen:
**EP - A - 0 062 916**
**EP - A - 0 063 340**
**EP - A - 0 063 343**

(73) Patentinhaber: **HOECHST AKTIENGESELLSCHAFT,
Postfach 80 03 20, D-6230 Frankfurt am Main 80 (DE)**

(72) Erfinder: **Dettbarn, Hans–Jürgen, Rehbocks Ecke 4,
D-3550 Marburg 9 (DE)**
Erfinder: **Zimmermann, Josef, Auf der Krautweide 11,
D-6231 Sulzbach (DE)**

## Beschreibung

Gegenstand der Erfindung ist ein nadelloses Injektionsgerät mit einer Kolbenpumpe für das zu injizierende Medium, die mit einem Antriebsmotor verbunden ist, an dessen Motorgehäuse ein Bedienungselement verschiebbar angeordnet ist.

Injektionsgeräte der genannten Art sind aus den europäischen Offenlegungsschriften 0 062 916 und 0 063 340 bekannt.

Es besteht die Aufgabe für diese bekannten Injektionsgeräte eine Möglichkeit zu schaffen, die Anzahl der Applikationsvorgänge zu zählen. Die Aufgabe wird durch ein Injektionsgerät gelöst, das dadurch gekennzeichnet ist, dass im Motorgehäuse ein Hubzähler angeordnet ist, an dessen freiem Wellenende eine Steuernocke befestigt ist, in die ein Steuerstift eingreift, der sich auf einer Steuerwalze abstützt, die im Bedienungselement für den Antriebsmotor angeordnet ist, wobei der Steuerstift, bei Verschiebung des Bedienungselementes, durch die Steuerwalze angehoben wird und dadurch auf die am Wellenende befestigte Steuernocke drückt, die die Hubzählerwelle dreht und somit den Hubzähler betätigt.

Im folgenden wird die Erfindung anhand von lediglich einen Ausführungsweg darstellenden Zeichnungen näher erläutert. Es zeigt:

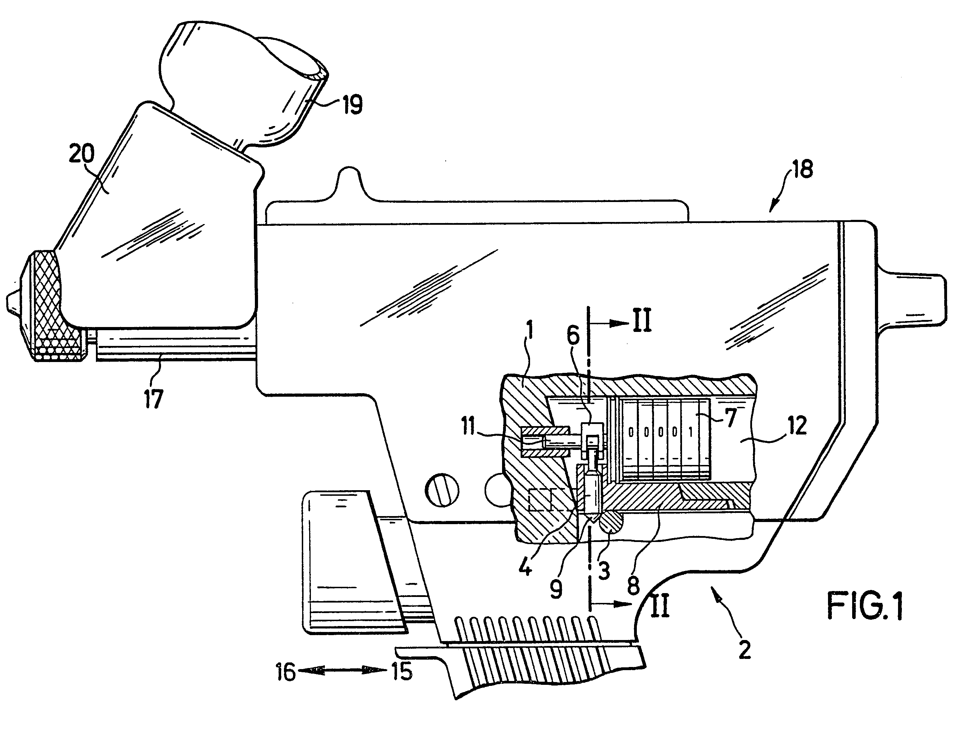

Fig. 1 eine Seitenansicht des Injektionsgerätes in geladenem Zustand teilweise geschnitten.

Fig. 2 den Schnitt II-II der Fig. 1.

Fig. 3 einen Ausschnitt «Z» der Fig. 1, bei dem der Steuerstift durch die Steuerwalze angehoben ist, das heisst, bei dem das Injektionsgerät entladen ist und

Fig. 4 den Schnitt IV-IV der Fig. 3.

Das nadellose Injektionsgerät besteht im wesentlichen aus einer Impfstoffpumpe 17 für das zu injizierende Medium, die mit einem Antriebsmotor 18 verbunden ist. Die Impfstoffpumpe 17 trägt die Einrichtung 20 für den Impfstoffbehälter 19. Das Bedienungselement 2 für den Antriebsmotor 18 ist beweglich am Motorgehäuse 1 angeordnet. In einer Ausnehmung 12 im Motorgehäuse 1 ist ein handelsüblicher Hubzähler 7 angeordnet, dessen Welle bauseitig mit einer Rückholfeder (nicht dargestellt) versehen ist. Das freie Wellenende 11 ist mit einer Steuernocke 6 versehen, die mit einer Schraube 5 auf der Welle gehalten wird. In die Steuernocke 6 greift ein Steuerstift mit seinem Zapfenende 10 ein, der in einer im Motorgehäuse 1 befindlichen Montageplatte 8 geführt wird. Das kegelförmig ausgebildete Ende 9 des Steuerstiftes 4 stützt sich auf einer Steuerwalze 3 ab, die im Bedienungselement 2 für den Antriebsmotor 18 angeordnet ist.

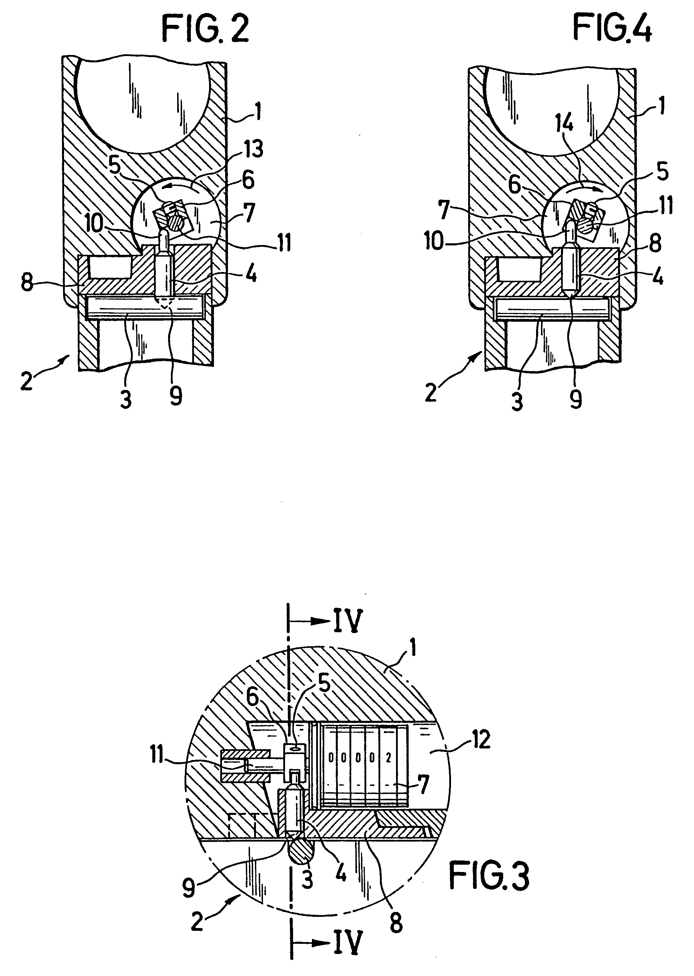

Die im Hubzähler eingebaute Rückholfeder dreht die Welle in Pfeilrichtung 13. Beim Spannen des Injektionsgerätes bewegt sich das Bedienungselement in Pfeilrichtung 15. Dadurch bewegt sich der Steuerstift unter der Wirkung der Rückholfeder des Hubzählers 7 in Richtung Steuerwalze 3 und die Welle 11 des Hubzählers führt eine Schwenkbewegung in Pfeilrichtung 13 (Fig. 2) aus. Beim Auslösen (Entspannen des Injektionsgerätes) der Injektion wird das Bedienungselement 2 in Pfeilrichtung 16 geschoben. Dabei hebt die Steuerwalze 3 den Steuerstift 4 an; das Zapfenende 10 drückt auf die am Wellenende 11 befestigte Steuernocke 6, die sich dadurch in Pfeilrichtung 14 dreht und die Hubzähleranzeige um einen Zähler weiterdreht.

## Patentanspruch

1. Nadelloses Injektionsgerät mit einer Kolbenpumpe (17) für das zu injizierende Medium, die mit einem Antriebsmotor (18) verbunden ist, an dessen Motorgehäuse (1) ein Bedienungselement (2) verschiebbar angeordnet ist, dadurch gekennzeichnet, dass im Motorgehäuse (1) ein Hubzähler (7) angeordnet ist, an dessen freiem Wellenende (11) eine Steuernocke (6) befestigt ist, in die ein Steuerstift (4) eingreift, der sich auf einer Steuerwalze (3) abstützt, die im Bedienungselement (2) für den Antriebsmotor (18) angeordnet ist, wobei der Steuerstift (4), bei Verschiebung des Bedienungselementes (2), durch die Steuerwalze (3) angehoben wird und dadurch auf die an Wellenende (11) befestigte Steuernocke (6) drückt, die die Hubzählerwelle (11) dreht und somit den Hubzähler (7) betätigt.

## Claim

A needleless injection instrument with a piston pump (17) for the medium to be injected, which pump is connected to a drive motor (18), on the motor housing (1), of which an operating element (2) is fitted displaceably, which comprises a stroke counter (7) which is arranged in the motor housing (1) and to the free shaft end (11) of which a control cam (6) is fixed which engages with a control pin (4) supported on a control roll (3) which is located in the operating element (2) for the drive motor (18), the control pin (4) being lifted by the control roll (3), when the operating element (2) is shifted, and thus acting on the control cam (6) which is fixed to the shaft end (11) and rotates the stroke counter shaft (11) and thus actuates the stroke counter (7).

## Revendication

Appareil d'injection sans aiguille comportant un piston de pompe (17) pour l'agent à injecter, qui est relié à un moteur d'entraînement (18) pourvu d'un carter de moteur (1) sur lequel est disposé un élément de manœuvre (2) avec possibilité de translation, caractérisé en ce qu'il est prévu dans le carter du moteur (1) un compteur de courses (7) comportant un arbre à l'extrémité libre (11) duquel est fixée une came de commande (6) dans laquelle s'engage une broche de commande (4) qui s'appuie sur un rouleau de commande (3) à qui est disposé dans l'élément de manœuvre (2) du moteur d'entraînement (18), la broche de commande (4), lors de la translation de l'élément de manœuvre (2), étant soulevée par le rouleau de commande (3), et s'appuyant ainsi contre la came de commande (6) fixée à l'extrémité (11) de l'arbre, cette came faisant tourner l'arbre (11) du compteur de courses et actionnant par conséquent ce compteur de courses (7).

0 169 494

FIG.1

# FIG. 2

# FIG. 4

# FIG. 3